# EUROPEAN PATENT SPECIFICATION

(11) **EP 1 855 665 B1**
(45) Date of publication and mention of the grant of the patent: **31.12.2008**
(21) Application number: 06736775.5
(22) Date of filing: 02.03.2006
(51) Int. Cl.: A61K 31/198, A61K 31/197, A61K 31/192, A61P 35/00, A61P 43/00, A61K 31/45

(54) **USE OF PHENYLACETYL-DERIVATIVES FOR THE MANUFACTURE OF A MEDICAMENT TO TREAT VON HIPPEL-LINDAU (VHL) DISEASE**
VERWENDUNG VON PHENYLACETYLDERIVATEN ZUR HERSTELLUNG EINES ARZNEIMITTELS ZUR BEHANDLUNG DER VON-HIPPEL-LINDAU-ERKRANKUNG
UTILISATION DE DERIVES DE PHENYLACETYLE POUR LA PRODUCTION D'UN MEDICAMENT EMPLOYE DANS LE TRAITEMENT DE LA MALADIE DE VON HIPPEL-LINDAU (VHL)

(30) Priority: 08.03.2005 US 659759 P
(43) Date of publication of application: 21.11.2007
(73) Proprietor: BURZYNSKI, Stanislaw R., Houston, TX 77042 (US)
(72) Inventor: BURZYNSKI, Stanislaw R., Houston, TX 77042 (US)
(74) Representative: Brants, Johan P.E.
(86) International application number: PCT/US2006/007511
(87) International publication number: WO 2006/096481

(56) References cited:
- US-A- 4 559 325
- US-A- 5 877 213
- US-A- 6 013 278
- US-A1- 2001 044 466

## Description

### FIELD OF THE INVENTION

The invention relates to treatments for *von Hippel-Lindau* disease (VHL) and, more specifically, to treating VHL using phenylacetyl-derivatives.

### BACKGROUND OF THE INVENTION

VHL (also known as Cerebroretinal angiomatosis) is comparatively rare genetic disorder characterized by the abnormal growth of tumors in certain parts of the body. The tumors of the central nervous system (CNS) are typically low grade and are comprised of a nest of abnormal blood vessels (called hemangioblastomas, or angiomas in the eye); pheochromocytoma; and endolymphatic sac tumors. Hemangioblastomas may develop in the brain, the retina of the eyes, and other areas of the nervous system and can cause death, when located in the brain stem. Other types of tumors develop in the adrenal glands, the kidneys (renal cell carcinoma occurs in about 40% of individuals with VHL), the pancreas, or other viscera. Symptoms of VHL vary among patients and depend on the size and location of the tumors. Symptoms may include headaches, problems with balance and walking (ataxia), dizziness, weakness of the limbs, vision problems, high blood pressure, seizures, and mental retardation. Cysts (fluid-filled sacs) and/or tumors (benign or cancerous) may develop around the hemangioblastomas and cause the symptoms listed above (Filling-Katz *et al*., 1991). Pheochromocytomas may be asymptomatic or may cause sustained or episodic hypertension. Endolymphatic sac tumors can cause hearing loss of varying severity, which is often a presenting symptom (Choo *et al*. 1994).

VHL is inherited in an autosomal dominant manner and testing of the von Hippel-Lindau tumor suppressor gene (*VHL* gene) detects mutations in virtually 100% of the individuals diagnosed with VHL. Molecular genetic analysis indicates that point mutations account for approximately 72% of *VHL* mutations, with partial or complete gene deletion accounting for the remaining 28% (Stolle *et al*.). It is estimated that 80% of individuals with VHL inherited it from an affected parent; while the remaining 20% have the disease as the result of a *de novo* gene mutation. The offspring of an affected individual have 50% risk of inheriting the disease. While the prevalence of VHL is not certain, recent data suggest that there may be thousands of people afflicted with this malady.

Additionally, the *VHL* gene (which resides on chromosome 3p25) is mutated or silenced in >50% of sporadic renal cell carcinomas (Kaelin, 2004) and there is evince that *VHL* gene mutation is a step in the progression to pancreatic islet cell tumors (Lott, *et al*., 2002)

Current used methods for treating VHL vary depending on the size and location of the tumor. They include standard surgical removal, gamma knife surgery, diathermy, xenon, laser, and cryocoagulation (Raja *et al*., 2004; Shingleton & Sewell, 2002). However, due to complexities including variations in the location and size of VHL-associated tumors, these modes of treatment are frequently impractical or ineffective.

Other treatments for VHL are currently being investigated and include the uses of vascular endothelial cell growth factor inhibitors (George and Kaelin, 2003; Aiello *et al*. 2002), other antiangiogenic drugs (*e*.*g*. Madhusudan, *et al*., 2004), and hypo-methylating agents, such as zebularine and 5-aza-2'-deoxycytidine (Alleman *et al*., 2004). However, none of these treatments have yet proven effective (for a more detailed summary of VHL, its believed causes, treatments, and prognosis see the VHL web site at www.geneclinics.org/profiles/vhl/). Thus, there exists a need for an effective treatment for VHL and its symptoms.

### SUMMARY OF THE INVENTION

Various embodiments of the invention provide an effective amount of pharmaceutical compositions comprising phenylacetylglutamine (or a pharmaceutically acceptable salt thereof), phenylacetylisoglutamine (or a pharmaceutically acceptable salt thereof), and/or phenylacetate (or a pharmaceutically acceptable salt thereof) for treating patients believed to be suffering from von Hippel-Lindau disease (VHL).

Other embodiments of the invention provide for the use of a composition comprising phenylacetylglutamine (or a pharmaceutically acceptable salt thereof), phenylacetylisoglutamine (or a pharmaceutically acceptable salt thereof), and/or phenylacetate (or a pharmaceutically acceptable salt thereof) for the manufacture of a medicament for treating a patient suspected of being afflicted with VHL.

### DEFINITIONS

The following definitions are provided in order to aid those skilled in the art in understanding the detailed description of the present invention.

As used herein the term "phenylacetyl-derivative" preferably refers to composition comprising phenylacetate and/or one or more analogs thereof that have been demonstrated to possess anti-cancer properties.

As used herein the term "pharmaceutically acceptable salt" means salts having the biological activity of the parent compound and lacking toxic activity at the selected administration level. Determination of whether a salt is pharmaceutically acceptable can be accomplished by methods known to those of skill in the art. By way of example only, pharmaceutically acceptable salts of phenylacetylglutamine, phenylacetylisoglutamine, and phenylacetic acid include inorganic sodium, potassium and ammonium salts, and organic diethanolamine, cyclohexylamine, and amino acid salts.

### ILLUSTRATIVE EMBODIMENTS OF THE INVENTION

Various embodiments of the instant invention provide compositions for use in treating a patient, preferably a human patient, believed to be afflicted with von Hippel-Lindau disease (VHL) (also known as von Hippel-Lindau syndrome). More specifically, these embodiments of the invention provide phenylacetyl-derivatives for use in treating a patient believed to be afflicted with VHL. In preferred aspects of these embodiments, a combination of (a) phenylacetylglutamine (or a pharmaceutically acceptable salt thereof), (b) phenylacetylisoglutamine (or a pharmaceutically acceptable salt thereof) and (c) phenylacetic acid (or a pharmaceutically acceptable salt thereof) is used to treat the patient(s). Hereafter, whenever the terms phenylacetylglutamine (PG), phenylacetylisoglutamine (isoPG), and phenylacetic acid (PN) are used, they should be viewed as encompassing both the acidic and pharmaceutically acceptable salt forms of the molecules.

Phenylacetylglutamine has a molecular weight of 264.28, its empirical formula is C₁₆H₁₆N₂O₄, and the structural formula provided below as Formula I.

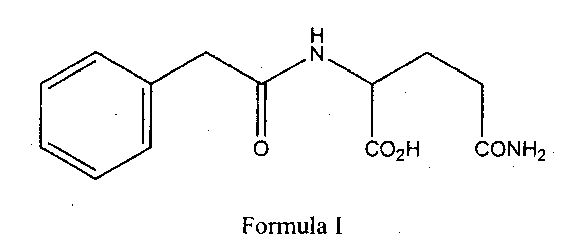

Phenylacetylisoglutamine has a molecular weight of 264.28; its empirical formula is C₁₆H₁₆N₂O₄, and the structural formula provided below as Formula II.

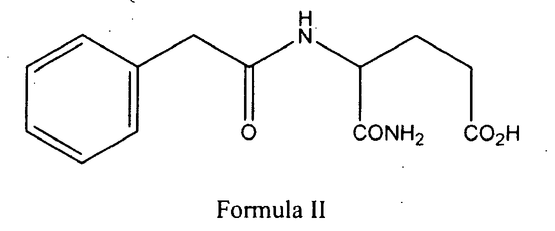

Phenylacetic acid has a molecular weight of 136.14, its empirical formula is C₈H₈O₂, and the structural formula is provided below as Formula III.

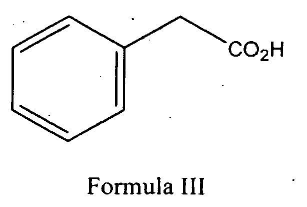

Various embodiments of the invention provide combinations of PG, isoPG, and/or PN for use in treating a patient diagnosed with VHL using. In certain aspects of these embodiments the PG, isoPG, and/or PN may be present in their acidic forms or as pharmaceutically acceptable salts.

In preferred aspects of these embodiments a composition comprising a combination of PG, isoPG, and PN is provided for use in treating the patient. Preferably, the composition comprises the sodium salts of PG; isoPG, and PN.

A more preferred embodiment of the invention comprises providing two pharmaceutical compositions for administration to the patient. According various aspects of this embodiment, the first pharmaceutical composition comprising a combination of PG and isoPG and the second pharmaceutical composition comprises PN and PG. Preferably each compound in the first and second pharmaceutical compositions is present in its sodium salt form. An even more preferred aspect of this embodiment provides that the first pharmaceutical composition comprises PG and isoPG present at a 4:1 ratio and that the second pharmaceutical composition comprises PN and PG present at a 4:1 ratio.

Various aspects of these embodiment provide the first and second pharmaceutical composition for administration by any appropriate and effective means known in the art, including: orally, intravenously or by some other parenteral means. In preferred aspects of this embodiment the first and second pharmaceutical composition are delivered orally and/or intravenously. In a particularly preferred aspect of this embodiment the first and second pharmaceutical composition are delivered intravenously as aqueous solutions. Various aspects of the embodiment provide for either sequential or simultaneous administration of the first and second pharmaceutical compositions.

In various aspects of this embodiment the first and second pharmaceutical compositions are provided as aqueous solutions for delivery, with the first pharmaceutical compositions comprising PG and isoPG present at a 4:1 ratio and the second pharmaceutical composition comprising PN and PG present at a 4:1 ratio. In particularly preferred aspects of this embodiment the combined concentration of PG and isoPG in the first pharmaceutical is from 50 to 400 mg/ml; preferably from 200 to 350 mg/ml; even more preferably 300 mg/ml. Similarly, preferred aspects of this embodiment provide for combined concentration of PN and PG in the second pharmaceutical composition of from 10 to 120 mg/ml; preferably, from 40 to 100 mg/ml; even more preferably 80 mg/ml.

Various aspects of this embodiment of the invention provide these first and second aqueous pharmaceutical compositions for use in intravenous delivery at infusion rates of from 20 to 400 ml/hour; preferably the infusion rates are from 50 to 250 ml/hour. Various aspects of this embodiment provide for infusion frequencies of from once every other day to 10 times, or more, daily. In a preferred aspect of this embodiment the infusions are given every 4 to 6 hours.

According to various aspects of this embodiment of the invention the first pharmaceutical composition (combination of PG and isoPG) is provided for delivery at a dose of from 0.5 to 25 g/kg/day; preferably from 5 to 20 g/kg/day. The second pharmaceutical composition is provided for delivery at a dose of from 0.02 to 1 g/kg/day; preferably from 0.1 to 0.4 g/kg/day.

In a particularly preferred embodiment a combination of two pharmaceutical compositions is provided for treatment of the patient diagnosed with VHL. The first pharmaceutical composition comprises an aqueous solution of sodium phenylacetylglutaminate and sodium phenylacetylisoglutaminate in a 4:1 ratio and at a combined concentration of 300 mg/ml. The second pharmaceutical composition comprises an aqueous solution of sodium phenylacetate and sodium phenylacetylglutaminate present in a 4:1 ratio at a concentration of 80 mg/ml. In various aspects of this embodiment of the invention the first and second pharmaceutical compositions are given sequentially every 4 to 6 hours to give a daily dosage of 10-20 g/kg of the first pharmaceutical composition and 0.2-0.4 g/kg of the second pharmaceutical composition. Various aspects of this invention provide for dosing occur at any effective frequency and for any effective/necessary duration. For example, daily dosing may continue for one week, two weeks, three weeks, one month, one year, or longer, as required. The decision as to the duration for treatment will depend on the individual patient's response and must would likely be make by the patient's physician after evaluation of the patient's response to the treatment.

Other embodiments of the invention provide an effective amount of PG, isoPG, and PN for use in the treatment of a patient diagnosed with VHL, and for oral administration. In preferred aspects of this embodiment, two pharmaceuticals are provided for use with the patient, the first pharmaceutical comprising PG and isoPG in a 4:1 ratio and the second pharmaceutical comprising PN and PG in a 4:1 ratio. The first and second pharmaceutical compositions may be orally delivered in any suitable form including: capsules, tablets, as a bolus, as a liquid solution or liquid suspension or using any suitable combination of these delivery forms. In various aspects of this embodiment treatment of the patient using oral delivery may be made prior to, concomitant with, or subsequent to treating the patient using another delivery form (*e*.*g*. intravenous delivery). One preferred aspect of this embodiment provides two pharmaceutical compositions for use in treating the patient in a first treatment regimen that comprises intravenous delivery of the two pharmaceutical compositions, the first pharmaceutical composition comprising PG and isoPG and the second pharmaceutical composition comprising PN and PG. The invention further provides third and fourth pharmaceutical compositions for oral administration to the patient in a second treatment regimen. At a different time, either prior to or subsequent to the first treatment regimen, the patient is treated with the second treatment regimen. The third pharmaceutical composition comprising PG and isoPG and the fourth pharmaceutical composition comprising PN and PG.

It is noted that in all aspects of the invention drawn to the use of PG, isoPG, and PN to treat VHL contemplate the use of the acidic form and/or a pharmaceutically acceptable salt form of each the compounds, with the sodium salt representing a preferred embodiment. Moreover, all possible combinations of the compounds, concentrations, delivery (*e*.*g*. infusion) rates, dosages, and treatment regimens described herein are part of the methods provided by the instant invention.

Other embodiments of the invention provide for the use of PG, isoPG, and/or PN for the manufacture of a pharmaceutical formulation effective for the treatment of von Hippel-Lindau disease. Various aspects of these embodiments provide for the use of the acidic form and/or a pharmaceutically acceptable salt for of each compound; if a salt form, the compound is preferably a sodium salt.

According to various aspects of this embodiment PG, isoPG, and PN are used to prepare two medicaments. The first medicament comprising PG and isoPG in a 4:1 ratio and the second medicament comprising PN and PG in a 4:1 ratio. In specific aspects of this embodiment, the medicament may be prepared for either oral (*e*.*g*. a bolus, capsule; tablet, suspension, or other suitable form) delivery or parenteral delivery (*e*.*g*. an sterile aqueous solution). In preferred aspects of this embodiment the first and second medicaments are aqueous solutions with the first medicament comprising PG and isoPG at a 4:1 ratio with a combined concentration of from 50-400 mg/ml; preferably 300 mg/ml. The second medicament comprises PN and PG in a 4:1 ratio with a combined concentration of from 10-120 mg/ml; preferably 80 mg/ml. In all aspects of this embodiment the medicaments are suitable for either simultaneous or sequential delivery at an infusion rate of from 20-400 ml/hour; preferably from 50-250 ml/hour.

In other embodiments of the invention the treatment of patients with VHL may comprise using a pro-drug and/or a pro-drug may be used to prepare a medicament to treat VHL. Suitable pro-drugs include 3-phenylacetylamino-2,6-piperidinedione (PP) (Formula IV) and phenylbutyrate (salt of phenylbutyric acid) (PB) (Formula V). 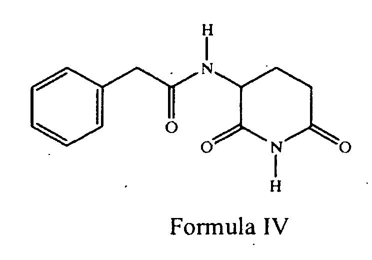 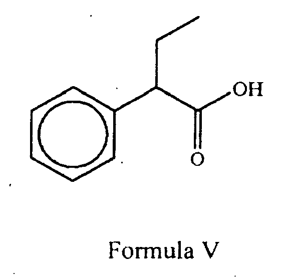 PP is metabolized in the small intestine to a combination of PG and isoPG. Similarly, PB is metabolized in the liver to a combination of PG and PN. Accordingly, various aspects of the instant inventions contemplate the use of pro-drugs that are metabolized to PG, isoPG, and/or PN, for treatment of patients believed to be suffering from VHL and for preparing such pro-drugs for the manufacture of a medicament effective for the treatment of VHL.

### EXAMPLES

The following examples are included to demonstrate preferred embodiments of the invention. It should be appreciated by those of skill in the art that the techniques disclosed in the examples which follow represent techniques discovered by the INVENTOR/S to function well in the practice of the invention, and thus can be considered to constitute preferred modes for its practice.

### Example 1: Treatment of 40-year old Caucasian VHL patient with phenylacetyl-derivatives

The patient, a 40-year old Caucasian male, developed ataxia, aphasia, and right facial nerve paralysis in January 2003. MRI and PET scans revealed progressive lesions in the brain stem, cerebellum and right internal capsule described as hemangioblastomas and a CT scan has shown a left renal cyst. A brain biopsy was not recommended because of associated risk.

In July 2003, molecular genetic study (MGS) for von Hippel-Lindau disease was performed at the Children's Hospital in Philadelphia (CHP) using whole blood as the tissue sample. The laboratory results of this testing indicate that the testing was performed as follows. First genomic DNA was extracted from peripheral blood. Next, the genomic DNA was digested with Eco RI and Ase I restriction endonucleases and the digested DNA was subjected to Southern blot analysis with hybridization to probes specific for the *VHL* gene (this was done to detect complete or partial deletions, following the methods previously described by Stolle *et al*., 1998. In this analysis the normal *VHL* gene fragment presents as a 9.7 kilobase DNA fragment.

Additionally, exons of the *VHL* gene were amplified by polymerase chain reaction (PCR) using primers indicated in Stolle *et al*., 1998 and then subjected to conformation sensitive gel electrophoresis (Ganguly *et al*., 1993) to screen for the presence of point mutations. The DNA fragments were sequenced and their sequences compared with controls.

The July 2003 MGS report indicates that the Southern blot analysis for the patient is consistent with the complete deletion of one allele of the *VHL* gene.

On July 30, 2003 the patient was admitted for intravenous administration of phenylacetyl-derivatives in accordance with Federal Drug Administration approved protocol BT-11. Pharmaceutical compositions comprising PG:isoPG (4:1) and PG:PN (1:4), respectively, were administered intravenously at a dosage of 10.72 g/kg/day and 0.33 g/kg/day respectively until January 25, 2004. From May 9, 2004 to July 13, 2004 the patient was given both compositions orally (0.15 g/kg/day). No corticosteroids were administered during the treatment with phenylacetyl-derivatives.

After 3 months of treatment with the phenylacetyl-derivatives the ataxia, aphasia, and facial paralysis were no longer evident. Moreover, a PET scan after 5 months of treatment and an MRI scan done at 11 and 15 months after the commencement of treatment, were within normal limits. Moreover, follow up MGS done at CHP after 3 months of treatment failed to identify a detectable deletion or point mutation in the patient's *VHL* gene.

### Example 2: Preparation and administration phenylacetyl-derivatives

Phenylacetyl-derivatives, specifically PG, isoPG, and PN, may be obtained by any means known to those of art skill in the art. For example see United States Patent number 6,258,849 (Burzynski). This patent describes the both the isolation of phenylacetyl-derivatives from human urine and the chemical synthesis of these derivatives.

For use in the treatment described in this example a phenylacetyl-derivative composition for injection as a sterile solution comprising sodium phenylacetylglutaminate (PG) and sodium phenylacetylisoglutaminate (isoPG) in an approximately 4:1 ratio, was prepared (PG/isoPG composition). The PG/isoPG composition was delivered intravenously at a concentration of ∼300 mg/ml (comprising ∼230-250 mg/ml of PG and ∼55-65 mg/ml of isoPG).

A PN/PG composition for injection is a sterile solution comprising sodium phenylacetate (PN) and PG in an approximately 4:1 ratio. The PN/PG composition was delivered intravenously at a concentration of ∼80 mg/ml (comprising ∼ 62-65 mg/ml of PN and ∼15-17. mg/ml of PG).

To prepare the PG/isoPG- and PN/PG-compositions for injection the PG, isoPG, and PN were dissolved in water and the pH was adjusted to ∼ 7.0 using sodium hydroxide.

Phenylacetyl-derivative compositions may be administered by any effective means including intravenously and orally. An exemplary dosage schedule for adults is as follows:
The individual injection may be given over a one-hour or longer period of time for a 70kg patient, depending on the patient's tolerance. If a patient experiences a chemical taste in his/her mouth during the injection the flow rate of the pump should be decreased to 200 ml/hour. If the patient continues to experience the chemical taste, the flow rate should be decreased to 150 ml/h, if the taste persists the rate should be decreased in 50 ml/h increments to 100 ml/h or until the taste dissipates.
On the first day of administration of phenylacetyl-derivatives the intravenous (IV) pump may be loaded with 240 ml (∼72g) of the PG/isoPG composition and 200 ml (∼16g) of the PN/PG composition. The patient is first given 10 ml of the PG/isoPG composition at an infusion rate of100 ml/h, followed in 15 minutes by 10 ml of the PN/PG composition at an infusion rate of 100 ml/h. Four hours from the beginning of the initial infusion, the patient is given 46 ml of the PG/isoPG composition at 250 ml/h followed by 38 ml of the PN/PG composition at 250 ml/h, the second infusion procedure is repeated every four hours.
Beginning with the second day the PG/isoPG-composition dose is increased daily in 20 ml increments until the highest tolerable or effective dose is reached, typically not exceeding 20 g/kg/day. Similarly, the PN/PG-composition dose is increased daily in 10 ml increments until the highest tolerable or effective dose is reached, typically not to exceed 0.4 g/kg/day.

### DOSAGE SCHEDULE FOR CHILDREN

The dosage schedule for children is different, depending on age. An exemplary dosage schedule is as follows.
On the first day of administration of phenylacetyl-derivatives, the flow rate of the pump is typically maintained at 25 ml/hour. Beginning with the second day of administration the individual injection will be given at 50 to 250 ml/hour, depending on the patient's age and tolerance. On the first day of administration the pump will be loaded with 60 ml of the PG/isoPG-composition and 60 ml of the PN/PG-composition. The volume of the injection will be approximately 10 ml every 4 hours (six times a day).
1. Six months to less than two years old: flow rate 50 ml/h
2. Two to less than four years old: flow rate 75 ml/h
3. Four to less than seven years old: flow rate 100 ml/h
4. Seven to less than 10 years old: flow rate 150 ml/h
5. 10 to less than 16 years old: flow rate 200 ml/h
6. 16 to 18 years old: flow rate 250 ml/h.
If the patient experiences a chemical taste in his/her mouth during the injection the flow rate will be decreased in 25 ml/h increments until the patient no longer experiences the chemical taste.
Beginning with the second day of treatment the phenylacetyl-derivatives may be administered as follows.
1. Less than 12 years old: the PG/isoPG-composition dose is increased daily, in 10 ml increments, until the highest tolerable or effective dose is reached, typically not exceeding 25 g/kg/day. The PN:PG-composition dose is increased daily, in 5 ml increments, until the highest tolerable or effective dose is reached, typically not exceeding 0.6 g/kg/day.
2. 12 years old to less than 16 years old: the PG/isoPG-composition dose is increased daily, in 20 ml increments, until the highest tolerable or effective dose is reached, typically not exceeding 25 g/kg/day. The PN/PG-composition dose is increased daily, in 5-10 ml increments, until the highest tolerable or effective dose is reached, typically not exceeding 0.6 g/kg/day.
3. 16 years old to less than 18 years old: PG/isoPG-composition dose is increased daily, in 20 ml increments, until the highest tolerable or effective dose is reached, typically not exceeding 25 g/kg/day. The PN/PG-composition dose is increased daily, in 10 ml increments, until the highest tolerable or effective dose is reached, typically not exceeding 0.6 g/kg/day.

### REFERENCES

Aiello LP, George DJ, Cahill MT, Wong JS, Cavallerano J, Hannah AL, Kaelin WG Jr (2002) Rapid and durable recovery of visual function in a patient with von hippel-lindau syndrome after systemic therapy with vascular endothelial growth factor receptor inhibitor su5416. Ophthalmology 109:1745-51.
Alleman W, Tabios R, Chandramouli G, Aprelikova O, Torres-Cabala C, Mendoza A, Rodgers C, Sopko N, Linehan W, Vasselli J (2004) The in vitro and in vivo effects of re-expressing methylated von Hippel-Lindau tumor suppressor gene in clear cell renal carcinoma with 5-Aza-2'-deoxycytidine. Clinical Cancer Res. 10:7011-7021.
Choo D, Shotland L, Mastroianni M, Glenn G, van Waes C, Linehan WM, Oldfield EH (2004) Endolymphatic sac tumors in von Hippel-Lindau disease. J Neurosurg 100:480-7
Filling-Katz MR, Choyke PL, Oldfield E, Chamas L, Patronas NJ, Glenn GM, Gorin MB, Morgan JK, Linehan WM, Seizinger BR, et al (1991) Central nervous system involvement in Von Hippel-Lindau disease. Neurology 41:41-6
Ganguly et al. (1993) Proc. Natl. Acad. Sci. USA 90:10325-10329.
George G and Kaelin W (2003) The von Hippel-Lindau protein, vascular endothelial growth factor, and kidney cancer. New England J. Medicine 349:419-421.
Kaelin, W.G. (2004) The Von-Hippel-Lindau tumor suppressor gene and kidney cancer. Clinical Cancer Research 10:6290s-6295s (supplement).
Lott S, Chandler D, Curley S, Foster C, El-Naggar A, Frazier M, Strong L, Lovell M, Killary A (2002) High frequency loss of heterozygosity in von Hippel-Lindau (VHL)-associated and sporadic pancreatic islet cell tumors: evidence for a stepwise mechanism for malignant conversion in VHL tumorigenesis. Cancer Research 62:1952-1955.
Madhusudan S, Deplanque G, Braybrooke JP, Cattell E, Taylor M, Price P, Tsaloumas MD, Moore N, Huson SM, Adams C, Frith P, Scigalla P, Harris AL (2004) Antiangiogenic therapy for von Hippel-Lindau disease. JAMA 291:943-4.
Raja D, Benz MS, Murray TG, Escalona-Benz EM, Markoe A (2004) Salvage external beam radiotherapy of retinal capillary hemangiomas secondary to von Hippel-Lindau disease: visual and anatomic outcomes. Ophthalmology 111:150-3.
Shingleton WB and Sewell PE Jr (2002) Percutaneous renal cryoablation of renal tumors in patients with von Hippel-Lindau disease. J Urol 167:1268-70.
Stolle C, Glenn G, Zbar B, Humphrey JS, Choyke P, Walther M, Pack S, Hurley K, Andrey C, Klausner R, Linehan WM (1998) Improved detection of germline mutations in the von Hippel-Lindau disease tumor suppressor gene. Hum Mutat 12:417-23.

## Claims

1. A composition for use in treating a patient diagnosed with von Hippel-Lindau disease, the composition comprising an effective amount of phenylacetylglutamine (PG), or a salt of PG; phenylacetylisoglutamine (isoPG), or a salt of isoPG; and/or phenylacetic acid (PN), or a salt of PN.

2. The composition of claim 1 comprising an effective amount of sodium phenylacetylglutaminate, sodium phenylacetylisoglutaminate, and sodium phenylacetate.

3. The composition of claim 1 comprising:
a) phenylacetylglutamine (PG), or a salt of PG, and phenylacetylisoglutamine (isoPG), or a salt of isoPG, or
b) phenylacetic acid (PN), or a salt of PN, and PG, or a salt of PG.

4. The composition of claim 3 comprising:
a) sodium phenylacetylglutaminate and sodium phenylacetylisoglutaminate in substantially a 4:1 ratio or
b) sodium phenylacetate and sodium phenylacetylglutaminate in substantially a 4:1 ratio.

5. The composition of any one of claims 1-4 which is formulated for oral administration.

6. The composition of any one of claims 1-4 which is formulated for parenteral or intravenous administration.

7. The composition of claims 3 or 4 wherein a) the combined concentration of sodium phenylacetylglutaminate and sodium phenylacetylisoglutaminate is between 50 and 400 mg/ml or b) the combined concentration of sodium phenylacetate and sodium phenylacetylglutaminate is between 10 and 120 mg/ml.

8. The composition of claim 7 wherein a) the combined concentration of sodium phenylacetylglutaminate and sodium phenylacetylisoglutaminate is about 300 mg/ml or b) the combined concentration of sodium phenylacetate and sodium phenylacetylglutaminate is about 80 mg/ml.

9. The composition of claims 7 or 8 which is suitable for administration intravenously at an infusion rate of from 20 ml/hr to 400 ml/hr.

10. The composition of claims 7 or 8 which is suitable for administration intravenously at an infusion rate of 50 ml/hour to 250 ml/hour.

11. Use of phenylacetylglutamine (PG), or a salt of PG; phenylacetylisoglutamine (isoPG), or a salt of isoPG; and/or phenylacetic acid (PN), or a salt of PN, for the manufacture of a medicament effective for the treatment of a patient suffering from von Hippel-Lindau disease.

12. The use of claim 11 wherein the medicament comprises an effective amount of sodium phenylacetylglutaminate, sodium phenylacetylisoglutaminate, and sodium phenylacetate.

13. The use of claim 11 wherein the medicament comprises:
a) phenylacetylglutamine (PG), or a salt of PG, and phenylacetylisoglutamine (isoPG), or a salt of isoPG, or
b) phenylacetic acid (PN), or a salt of PN, and PG, or a salt of PG.

14. The use of claim 13 wherein the medicament comprises:
a) sodium phenylacetylglutaminate and sodium phenylacetylisoglutaminate in substantially a 4:1 ratio or
b) sodium phenylacetate and sodium phenylacetylglutaminate in substantially a 4:1 ratio.

15. The use of any of claims 11-14 wherein the medicament is suitable for administering to the patient orally.

16. The use of any one of claims 11-14 wherein the medicament is suitable for administering to the patient parenterally or intravenously.

17. The use of claims 13 or 14 wherein the medicament comprises:
a) a combined concentration of sodium phenylacetylglutaminate and sodium phenylacetylisoglutaminate between 50 mg/ml and 400 mg/ml or
b) a combined concentration of sodium phenylacetate and sodium phenylacetylglutaminate between 10 mg/ml and 120 mg/ml.

18. The use of claim 17 wherein the medicament comprises:
a) the combined concentration of sodium phenylacetylglutaminate and sodium phenylacetylisoglutaminate of 300 mg/ml or
b) the combined concentration of sodium phenylacetate and sodium phenylacetylglutaminate of 80 mg/ml.

19. The use of claims 17 or 18 wherein the medicament is suitable for administration intravenously at an infusion rate of from 20 ml/hr to 400 ml/hr.

20. The use of claims 17 or 18 wherein the medicament is suitable for administration intravenously at an infusion rate of 50 ml/hour to 250 ml/hour.

21. A composition for use in treating a human patient diagnosed with von Hippel-Lindau disease, the composition comprising in tandem:
a first pharmaceutical composition and a second pharmaceutical composition for intravenous administration;
a) wherein the first pharmaceutical composition comprises an aqueous solution of sodium phenylacetylglutaminate and sodium phenylacetylisoglutaminate present at a 4:1 ratio and having a combined concentration of from about 50 to 400 mg/ml;
b) wherein the second pharmaceutical composition comprises an aqueous solution of sodium phenylacetate and sodium phenylacetylglutaminate present at a 4:1 ratio and having a combined concentration of from 10 to 120 mg/ml;
wherein the first and second pharmaceutical are designed to be administered at an infusion rate of from 20 to 400 ml/hour.

22. The composition of claim 21 wherein the concentration of the first pharmaceutical composition is 300 mg/ml; the concentration of the second pharmaceutical composition is 80 mg/ml and the infusion rate for the first and second pharmaceutical compositions is 250 ml/hour.

23. Use of a composition for the manufacture of a medicament for treating a human patient diagnosed with von Hippel-Lindau disease, the composition comprising in tandem:
a first pharmaceutical composition and a second pharmaceutical composition for intravenous administration;
a) wherein the first pharmaceutical composition comprises an aqueous solution of sodium phenylacetylglutaminate and sodium phenylacetylisoglutaminate present at a 4:1 ratio and having a combined concentration of from about 50 to 400 mg/ml;
b) wherein the second pharmaceutical composition comprises an aqueous solution of sodium phenylacetate and sodium phenylacetylglutaminate present at a 4:1 ratio and having a combined concentration of from 10 to 120 mg/ml;
wherein the first and second pharmaceutical are designed to be administered at an infusion rate of from 20 to 400 ml/hour.

24. The use of claim 23 wherein the concentration of the first pharmaceutical composition is about 300 mg/ml; the concentration of the second pharmaceutical composition is about 80 mg/ml and the infusion rate for the first and second pharmaceutical compositions is about 250 ml/hour.

## Patentansprüche

1. Zusammensetzung zur Verwendung bei der Behandlung eines Patienten, bei dem die von-Hippel-Lindau'sche Krankheit diagnostiziert wurde, wobei die Zusammensetzung eine wirksame Menge an Phenylacetylglutamin (PG), oder eines Salzes von PG; Phenylacetylisoglutamin (isoPG), oder eines Salzes von isoPG; und Phenylessigsäure (PN), oder eines Salzes von PN umfasst.

2. Zusammensetzung nach Anspruch 1, die eine wirksame Menge an Natriumphenylacetylglutaminat, Natriumphenylacetylisoglutaminat und Natriumphenylacetat umfasst.

3. Zusammensetzung nach Anspruch 1, umfassend:
a) Phenylacetylglutamin (PG), oder ein Salz von PG, und Phenylacetylisoglutamin (isoPG), oder ein Salz von isoPG, oder
b) Phenylessigsäure (PN), oder ein Salz von PN, und PG, oder ein Salz von PG.

4. Zusammensetzung nach Anspruch 3, umfassend:
a) Natriumphenylacetylglutaminat und Natriumphenylacetylisoglutaminat in einem im Wesentlichen 4:1-Verhältnis, oder
b) Natriumphenylacetat und Natriumphenylacetylglutaminat in einem im Wesentlichen 4:1-Verhältnis.

5. Zusammensetzung nach einem der Ansprüche 1 bis 4, die zur oralen Verabreichung formuliert ist.

6. Zusammensetzung nach einem der Ansprüche 1 bis 4, die zur parenteralen oder intravenösen Verabreichung formuliert ist.

7. Zusammensetzung nach den Ansprüchen 3 oder 4, wobei a) die Gesamtkonzentration an Natriumphenylacetylglutaminat und Natriumphenylacetylisoglutaminat zwischen 50 und 400 mg/ml liegt, oder b) die Gesamtkonzentration an Natriumphenylacetat und Natriumphenylacetylglutaminat zwischen 10 und 120 mg/ml liegt.

8. Zusammensetzung nach Anspruch 7, wobei a) die Gesamtkonzentration an Natriumphenylacetylglutaminat und Natriumphenylacetylisoglutaminat etwa 300 mg/ml beträgt, oder b) die Gesamtkonzentration an Natriumphenylacetat und Natriumphenylacetylglutaminat etwa 80 mg/ml beträgt.

9. Zusammensetzung nach den Ansprüchen 7 oder 8, die sich zur intravenösen Verabreichung bei einer Infusionsgeschwindigkeit von 20 ml/Std. bis 400 ml/Std. eignet.

10. Zusammensetzung nach den Ansprüchen 7 oder 8, die sich zur intravenösen Verabreichung bei einer Infusionsgeschwindigkeit von 50 ml/Std. bis 250 ml/Std. eignet.

11. Verwendung von Phenylacetylglutamin (PG), oder einem Salz von PG; Phenylacetylisoglutamin (isoPG, oder einen Salz von isoPG; und Phenylessigsäure (PN), oder einem Salz von PN zur Herstellung eines Medikaments, das bei der Behandlung eines Patienten wirksam ist, der an der von-Hippel-Lindau'schen Krankheit leidet.

12. Verwendung nach Anspruch 11, wobei das Medikament eine wirksame Menge an Natriumphenylacetylglutaminat, Natriumphenylacetylisoglutaminat und Natriumphenylacetat umfasst.

13. Verwendung nach Anspruch 11, wobei das Medikament umfasst:
a) Phenylacetylglutamin (PG), oder ein Salz von PG, und Phenylacetylisoglutamin (isoPG), oder ein Salz von isoPG, oder
b) Phenylessigsäure (PN), oder ein Salz von PN, und PG, oder ein Salz von PG.

14. Verwendung nach Anspruch 13, wobei das Medikament umfasst:
a) Natriumphenylacetylglutaminat und Natriumphenylacetylisoglutaminat in einem im Wesentlichen 4:1-Verhältnis, oder
b) Natriumphenylacetat und Natriumphenylacetylglutaminat in einem im Wesentlichen 4:1-Verhältnis.

15. Verwendung nach einem der Ansprüche 11 bis 14, wobei sich das Medikament zur oralen Verabreichung an den Patienten eignet.

16. Verwendung nach irgendeinem der Ansprüche 11 bis 14, wobei sich das Medikament zur parenteralen oder intravenösen Verabreichung an den Patienten eignet.

17. Verwendung nach den Ansprüchen 13 oder 14, wobei das Medikament umfasst:
a) eine Gesamtkonzentration an Natriumphenylacetylglutaminat und Natriumphenylacetylisoglutaminat zwischen 50 mg/ml und 400 mg/ml oder
b) eine Gesamtkonzentration an Natriumphenylacetat und Natriumphenylacetylglutaminat zwischen 10 mg/ml und 120 mg/ml.

18. Verwendung nach Anspruch 17, wobei das Medikament umfasst:
a) die Gesamtkonzentration an Natriumphenylacetylglutaminat und Natriumphenylacetylisoglutaminat von 300 mg/ml oder
b) die Gesamtkonzentration an Natriumphenylacetat und Natriumphenylacetylglutaminat von 80 mg/ml.

19. Verwendung nach den Ansprüchen 17 oder 18, wobei sich das Medikament zur intravenösen Verabreichung bei einer Infusionsgeschwindigkeit von 20 ml/Std. bis 400 ml/Std. eignet.

20. Verwendung nach den Ansprüchen 17 oder 18, wobei sich das Medikament zur intravenösen Verabreichung bei einer Infusionsgeschwindigkeit von 50 ml/Std. bis 250 ml/Std. eignet.

21. Zusammensetzung zur Verwendung bei der Behandlung eines menschlichen Patienten, bei dem die von-Hippel-Lindau'sche Krankheit diagnostiziert wurde, wobei die Zusammensetzung gemeinsam umfasst:
eine erste pharmazeutische Zusammensetzung und eine zweite pharmazeutische Zusammensetzung zur intravenösen Verabreichung;
a) wobei die erste pharmazeutische Zusammensetzung eine wässrige Lösung aus Natriumphenylacetylglutaminat und Natriumphenylacetylisoglutaminat umfasst, die in einem 4:1-Verhältnis vorhanden sind, und eine Gesamtkonzentration von etwa 50 bis 400 mg/ml aufweist;
b) wobei die zweite pharmazeutische Zusammensetzung eine wässrige Lösung aus Natriumphenylacetat und Natriumphenylacetylglutaminat umfasst, die in einem 4:1-Verhältnis vorhanden sind, und eine Gesamtkonzentration von 10 bis 120 mg/ml aufweist;
wobei das erste und das zweite Arzneimittel konzipiert sind, um bei einer Infusionsgeschwindigkeit von 20 bis 400 ml/Std. verabreicht zu werden.

22. Zusammensetzung nach Anspruch 21, wobei die Konzentration der ersten pharmazeutischen Zusammensetzung 300 mg/ml beträgt; die Konzentration der zweiten pharmazeutischen Zusammensetzungen 80 mg/ml beträgt und die Infusionsgeschwindigkeit für die erste und zweite pharmazeutische Zusammensetzung 250 ml/Std. beträgt.

23. Verwendung einer Zusammensetzung zur Herstellung eines Medikaments zur Behandlung eines menschlichen Patienten, bei dem die von-Hippel-Lindau'sche Krankheit diagnostiziert wurde, wobei die Zusammensetzung gemeinsam umfasst:
eine erste pharmazeutische Zusammensetzung und eine zweite pharmazeutische Zusammensetzung zur intravenösen Verabreichung;
a) wobei die erste pharmazeutische Zusammensetzung eine wässrige Lösung aus Natriumphenylacetylglutaminat und Natriumphenylacetylisoglutaminat umfasst, die in einem 4:1-Verhältnis vorhanden sind, und eine Gesamtkonzentration von etwa 50 bis 400 mg/ml aufweist;
b) wobei die zweite pharmazeutische Zusammensetzung eine wässrige Lösung aus Natriumphenylacetat und Natriumphenylacetylglutaminat umfasst, die in einem 4:1-Verhältnis vorhanden sind, und eine Gesamtkonzentration von 10 bis 120 mg/ml aufweist;
wobei das erste und das zweite Arzneimittel konzipiert sind, um bei einer Infusionsgeschwindigkeit von 20 bis 400 ml/Std. verabreicht zu werden.

24. Verwendung nach Anspruch 23, wobei die Konzentration der ersten pharmazeutischen Zusammensetzung etwa 300 mg/ml beträgt; die Konzentration der zweiten pharmazeutischen Zusammensetzungen etwa 80 mg/ml beträgt und die Infusionsgeschwindigkeit für die erste und zweite pharmazeutische Zusammensetzung etwa 250 ml/Std. beträgt.

## Revendications

1. Composition à utiliser dans le traitement d'un patient diagnostiqué avec la maladie de von Hippel-Lindau, la composition comprenant une quantité efficace de phénylacétylglutamine (PG), ou un sel de PG ; de phénylacétylisoglutamine (isoPG), ou un sel d'isoPG ; et/ou d'acide phénylacétique (PN), ou un sel de PN.

2. Composition selon la revendication 1, comprenant une quantité efficace de phénylacétylglutaminate de sodium, phénylacétylisoglutaminate de sodium, et phénylacétate de sodium.

3. Composition selon la revendication 1, comprenant :
a) de la phénylacétylglutamine (PG), ou un sel de PG, et de la phénylacétylisoglutamine (isoPG), ou un sel d'isoPG, ou
b) de l'acide phénylacétique (PN), ou un sel de PN, et PG, ou un sel de PG.

4. Composition selon la revendication 3, comprenant :
a) du phénylacétylglutaminate de sodium et du phénylacétylisoglutaminate de sodium dans un rapport essentiellement de 4 : 1 ou
b) du phénylacétate de sodium et du phénylacétylglutaminate de sodium dans un rapport essentiellement de 4 : 1.

5. Composition selon l'une quelconque des revendications 1 à 4, qui est formulée pour une administration orale.

6. Composition selon l'une quelconque des revendications 1 à 4, qui est formulée pour une administration parentérale ou intraveineuse.

7. Composition selon la revendication 3 ou la revendication 4, dans laquelle a) la concentration combinée de phénylacétylglutaminate de sodium et de phénylacétylisoglutaminate de sodium est entre 50 et 400 mg/ml ou b) la concentration combinée de phénylacétate de sodium et de phénylacétylglutaminate de sodium est entre 10 et 120 mg/ml.

8. Composition selon la revendication 7, dans laquelle a) la concentration combinée de phénylacétylglutaminate de sodium et de phénylacétylisoglutaminate de sodium est environ de 300 mg/ml ou b) la concentration combinée de phénylacétate de sodium et de phénylacétylglutaminate de sodium est environ de 80 mg/ml.

9. Composition selon la revendication 7 ou la revendication 8, qui est appropriée pour une administration intraveineuse à une vitesse de perfusion de 20 ml/h à 400 ml/h.

10. Composition selon la revendication 7 ou la revendication 8, qui est appropriée pour une administration intraveineuse à une vitesse de perfusion de 50 ml/h à 250 ml/h.

11. Utilisation de phénylacétylglutamine (PG), ou un sel de PG ; de phénylacétylisoglutamine (isoPG), ou un sel d'isoPG ; et/ou d'acide phénylacétique (PN), ou un sel de PN, pour la fabrication d'un médicament efficace dans le traitement d'un patient souffrant de la maladie de von Hippel-Lindau.

12. Utilisation selon la revendication 11, dans laquelle le médicament comprend une quantité efficace de phénylacétylglutaminate de sodium, phénylacétylisoglutaminate de sodium, et phénylacétate de sodium.

13. Utilisation selon la revendication 11, dans laquelle le médicament comprend :
a) de la phénylacétylglutamine (PG), ou un sel de PG, et de la phénylacétylisoglutamine (isoPG), ou un sel d'isoPG, ou
b) de l'acide phénylacétique (PN), ou un sel de PN, et PG, ou un sel de PG.

14. Utilisation selon la revendication 13, dans laquelle le médicament comprend :
a) du phénylacétylglutaminate de sodium et du phénylacétylisoglutaminate de sodium dans un rapport essentiellement de 4 : 1 ou
b) du phénylacétate de sodium et du phénylacétylglutaminate de sodium dans un rapport essentiellement de 4 : 1.

15. Utilisation selon l'une quelconque des revendications 11 à 14, dans laquelle le médicament est approprié pour une administration orale au patient.

16. Utilisation selon l'une quelconque des revendications 11 à 14, dans laquelle le médicament est approprié pour une administration parentérale ou intraveineuse au patient.

17. Utilisation selon la revendication 13 ou la revendication 14, dans laquelle le médicament comprend :
a) une concentration combinée de phénylacétylglutaminate de sodium et de phénylacétylisoglutaminate de sodium entre 50 mg/ml et 400 mg/ml, ou
b) une concentration combinée de phénylacétate de sodium et de phénylacétylglutaminate de sodium entre 10 mg/ml et 120 mg/ml.

18. Utilisation selon la revendication 17, dans laquelle le médicament comprend :
a) la concentration combinée de phénylacétylglutaminate de sodium et de phénylacétylisoglutaminate de sodium de 300 mg/ml ou
b) la concentration combinée de phénylacétate de sodium et de phénylacétylglutaminate de sodium de 80 mg/ml.

19. Utilisation selon la revendication 17 ou la revendication 18, dans laquelle le médicament est approprié pour une administration intraveineuse à une vitesse de perfusion de 20 ml/h à 400 ml/h.

20. Utilisation selon la revendication 17 ou la revendication 18, dans laquelle le médicament est approprié pour une administration intraveineuse à une vitesse de perfusion de 50 ml/h à 250 ml/h.

21. Composition à utiliser dans le traitement d'un patient humain diagnostiqué avec la maladie de von Hippel-Lindau, la composition comprenant en tandem :
une première composition pharmaceutique et une deuxième composition pharmaceutique pour une administration intraveineuse ;
a) dans laquelle la première composition pharmaceutique comprend une solution aqueuse de phénylacétylglutaminate de sodium et de phénylacétylisoglutaminate de sodium présents dans un rapport de 4 : 1 et présentant une concentration combinée d'environ 50 à 400 mg/ml ;
b) dans laquelle la deuxième composition pharmaceutique comprend une solution aqueuse de phénylacétate de sodium et de phénylacétylglutaminate de sodium présents dans un rapport de 4 : 1 et présentant une concentration combinée de 10 à 120 mg/ml ;
dans laquelle la première et la deuxième compositions pharmaceutiques sont conçues pour être administrées à une vitesse de perfusion de 20 à 400 ml/heure.

22. Composition selon la revendication 21, dans laquelle la concentration de la première composition pharmaceutique est de 300 mg/ml ; la concentration de la deuxième composition pharmaceutique est de 80 mg/ml et la vitesse de perfusion pour la première et la deuxième compositions pharmaceutiques est de 250 ml/heure.

23. Utilisation d'une composition pour la fabrication d'un médicament pour le traitement d'un patient humain diagnostiqué avec la maladie de von Hippel-Lindau, la composition comprenant en tandem :
une première composition pharmaceutique et une deuxième composition pharmaceutique pour une administration intraveineuse ;
a) dans laquelle la première composition pharmaceutique comprend une solution aqueuse de phénylacétylglutaminate de sodium et de phénylacétylisoglutaminate de sodium présents dans un rapport de 4 : 1 et présentant une concentration combinée d'environ 50 à 400 mg/ml ;
b) dans laquelle la deuxième composition pharmaceutique comprend une solution aqueuse de phénylacétate de sodium et de phénylacétylglutaminate de sodium présents dans un rapport de 4 : 1 et présentant une concentration combinée de 10 à 120 mg/ml ;
dans laquelle la première et la deuxième compositions pharmaceutiques sont conçues pour être administrées à une vitesse de perfusion de 20 à 400 ml/heure.

24. Utilisation selon la revendication 23, dans laquelle la concentration de la première composition pharmaceutique est environ de 300 mg/ml ; la concentration de la deuxième composition pharmaceutique est environ de 80 mg/ml et la vitesse de perfusion pour la première et la deuxième compositions pharmaceutiques est environ de 250 mg/ml.
